(19) **Europäisches Patentamt / European Patent Office / Office européen des brevets**

(11) **EP 4 306 119 A1**

(12) **EUROPEAN PATENT APPLICATION**
published in accordance with Art. 153(4) EPC

(43) Date of publication:
**17.01.2024 Bulletin 2024/03**

(21) Application number: **22767137.7**

(22) Date of filing: **08.03.2022**

(51) International Patent Classification (IPC):
**A61K 35/74** (2015.01)  **A23L 33/135** (2016.01)
**A61P 1/04** (2006.01)  **A61P 3/10** (2006.01)
**A61P 17/00** (2006.01)  **A61P 19/02** (2006.01)
**A61P 25/00** (2006.01)  **A61P 29/00** (2006.01)
**A61P 31/00** (2006.01)  **A61P 37/02** (2006.01)
**A61P 37/06** (2006.01)  **A61P 37/08** (2006.01)
**A61P 43/00** (2006.01)  **C12N 1/20** (2006.01)

(52) Cooperative Patent Classification (CPC):
**A23L 33/135; A61K 35/74; A61P 1/04; A61P 3/10;**
**A61P 17/00; A61P 19/02; A61P 25/00;**
**A61P 29/00; A61P 31/00; A61P 37/02;**
**A61P 37/06; A61P 37/08; A61P 43/00; C12N 1/20**

(86) International application number:
**PCT/JP2022/009997**

(87) International publication number:
**WO 2022/191183 (15.09.2022 Gazette 2022/37)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **08.03.2021 JP 2021036484**

(71) Applicants:
• **Juntendo Educational Foundation**
  **Tokyo 113-8421 (JP)**
• **Kyowa Kirin Co., Ltd.**
  **Tokyo 100-0004 (JP)**
• **Kyowa Hakko Bio Co., Ltd.**
  **Tokyo 100-8185 (JP)**

(72) Inventors:
• **ISHIKAWA Dai**
  **Tokyo 113-8421 (JP)**
• **KOIZUMI Satoshi**
  **Tokyo 100-8185 (JP)**
• **YAMASHITA Makoto**
  **Tokyo 100-8185 (JP)**
• **YAMAZAKI Fuhito**
  **Tokyo 100-8185 (JP)**
• **HONMA Nakayuki**
  **Tokyo 100-0004 (JP)**
• **URAKAWA Itaru**
  **Tokyo 100-0004 (JP)**
• **IWAMOTO Susumu**
  **Tokyo 100-0004 (JP)**

(74) Representative: **Vossius & Partner**
**Patentanwälte Rechtsanwälte mbB**
**Siebertstraße 3**
**81675 München (DE)**

(54) **COMPOSITION AND IMPROVING AGENT HAVING INFLAMMATION-SUPPRESSING ACTION**

(57)  The present invention relates to a composition which contains an intestinal bacterium belonging to the genus Veillonella and having an inflammation reducing activity, or a substance derived from the intestinal bacterium and having an inflammation reducing activity and which has at least one activity selected from an anti-inflammatory activity, an immunoregulation activity, an epithelial barrier restoring activity, an IL-10-inducing activity, and an IL-22-inducing activity, and to an ameliorating agent for an inflammatory disease, an autoimmune disease, or an infectious disease containing the intestinal bacterium and the substance derived from the intestinal bacterium and having an inflammation reducing activity.

**(Cont. next page)**

EP 4 306 119 A1

## FIG. 1

**Description**

TECHNICAL FIELD

The present invention relates to a composition containing an intestinal bacterium having an inflammation reducing activity or a substance derived from the intestinal bacterium and having an inflammation reducing activity, and an ameliorating agent for an inflammatory disease, an autoimmune disease, or an infectious disease, which contains the intestinal bacterium or the substance.

BACKGROUND ART

Inflammatory bowel diseases such as ulcerative colitis (UC) and Crohn's disease are "diseases in which exacerbation and remission are repeated and which cannot be cured". Although a therapeutic effect is dramatically improved with the appearance of a novel drug therapy, there is also a risk of ineffective cases or a side effect derived from a drug. From such a situation, there is a demand for a fundamental treatment for the inflammatory bowel diseases, which has less side effects with different action mechanisms.

The fecal microbiota transplantation has attracted attention because of exhibiting a very high response rate in relapsing Clostridium difficile infectious enteritis, and has been already recognized as a standard therapy for the disease in Europe and the United States because of less side effects. On the other hand, regarding the common fecal microbiota transplantation for inflammatory bowel diseases, it has been reported that there is a large difference in therapeutic effects depending on the methods, and thus it is required to clarify the cause of effectiveness and establish a better therapeutic method.

In the fecal microbiota transplantation for ulcerative colitis, the present inventors have conducted clinical research of fecal microbiota transplantation used in combination with an antibiotic therapy, aiming at more strongly ameliorating the abnormality of intestinal flora. In the clinical research, as a result of analyzing the clinical data and intestinal bacterium data of about 180 UC patients, it has been found that, in the case for which the effect is obtained in four weeks after the fecal microbiota transplantation, a ratio of Bacteroidetes is significantly recovered, and the recovery of Bacteroidetes is correlated with an endoscopic score representing a state of an illness of ulcerative colitis (Non Patent Literature 1).

On the other hand, regarding the inflammation reducing activity generated by the intestinal bacterium, a mixture of Clostridium bacteria having a Treg-inducing activity for reducing immune response has been known (Non Patent Literatures 2 and 3). Further, in mouse Crohn's disease models, it has been known that some of the strains of Bacteroides thetaiotaomicron are effective in increasing the percentage of Treg (Patent Literature 1 and Non Patent Literature 4), and that Bacteroides ovatus is effective in ameliorating inflammation and the like (Non Patent Literature 5).

CITATION LIST

Patent Literature

Patent Literature 1: WO2016/102950

Non Patent Literature

Non Patent Literature 1: Ishikawa et al., Inflamm Bowel Dis., 2018, 24:2590-2598
Non Patent Literature 2: Atarashi et al., Science, 2011, 331:337-341
Non Patent Literature 3: Atarashi et al., Nature, 2013, 500:232-238
Non Patent Literature 4: Delday et al., Inflamm Bowel Dis. 2019, 25:85-96
Non Patent Literature 5: Ihekweazu et al., GUT MICROBES, 2019, 10:504-520

SUMMARY OF INVENTION

Technical Problem

As described above, regarding the inflammatory bowel diseases, there is a demand for a fundamental treatment having less side effects with different action mechanisms. Regarding the therapeutic effect of the fecal microbiota transplantation in the related art, there is a report indicating effectiveness, but it cannot be said that the therapeutic effect has been established. It is required to clarify the cause of effectiveness and establish a better therapeutic method. However, an intestinal bacterium derived from a donor whose inflammatory bowel disease is recognized to be ameliorated by the fecal microbiota transplantation in humans and exhibiting an inflammation reducing activity has not been known.

Therefore, an object of the present invention is to provide a composition containing an intestinal bacterium having less side effects and an excellent inflammation reducing activity or a substance derived from the intestinal bacterium and having an inflammation reducing activity, and an ameliorating agent for an inflammatory disease, an autoimmune disease, or an infectious disease, which contains the intestinal bacterium or the substance derived from the intestinal bacterium and having an inflammation reducing activity.

SOLUTION TO PROBLEM

The present inventors have analyzed intestinal bacterium data of a patient with ulcerative colitis to find that a high response rate of ulcerative colitis is correlated with a change in intestinal flora, and have analyzed species of intestinal bacteria in effective examples after a long period to find species of bacteria involved in improvement and maintenance of therapeutic effects. The present invention completed based on these findings is as follows.

1. A composition containing an intestinal bacterium belonging to the genus Veillonella and having an inflammation reducing activity or a substance derived from the intestinal bacterium and having an inflammation reducing activity, the composition having at least one activity selected from an anti-inflammatory activity, an immunoregulation activity, an epithelial barrier restoring activity, an IL-10-inducing activity, and an IL-22-inducing activity.

2. The composition according to the above 1, in which the intestinal bacterium is Veillonella sp. (JKB232V).

3. The composition according to the above 1 or 2, further containing an intestinal bacterium belonging to Bacteroidetes and having an inflammation reducing activity, or a substance derived from the intestinal bacterium and having an inflammation reducing activity.

4. The composition according to the above 3, in which the intestinal bacterium belonging to Bacteroidetes is Alistipes putredinis, Bacteroides cellulosilyticus, Bacteroides intestinalis, or Bacteroides dorei.

5. The composition according to the above 4, in which the above Alistipes putredinis, Bacteroides cellulosilyticus, Bacteroides intestinalis, and Bacteroides dorei are Alistipes putredinis (JKB232A), Bacteroides cellulosilyticus (JKB161), Bacteroides intestinalis (JKB231), and Bacteroides dorei (JKB233), respectively.

6. An ameliorating agent for an inflammatory disease, an autoimmune disease, and an infectious disease, which contains an intestinal bacterium belonging to the genus Veillonella and having an inflammation reducing activity or a substance derived from the intestinal bacterium and having an inflammation reducing activity, and which has at least one activity selected from an anti-inflammatory activity, an immunoregulation activity, an epithelial barrier restoring activity, an IL-10-inducing activity, and an IL-22-inducing activity.

7. The ameliorating agent according to the above 6, in which the intestinal bacterium is Veillonella sp. (JKB232V).

8. The ameliorating agent according to the above 6 or 7, further containing an intestinal bacterium belonging to Bacteroidetes and having an inflammation reducing activity, or a substance derived from the intestinal bacterium and having an inflammation reducing activity.

9. The ameliorating agent according to the above 8, in which the intestinal bacterium belonging to Bacteroidetes is Alistipes putredinis, Bacteroides cellulosilyticus, Bacteroides intestinalis, or Bacteroides dorei.

10. The ameliorating agent according to the above 9, in which the above Alistipes putredinis, Bacteroides cellulosilyticus, Bacteroides intestinalis, and Bacteroides dorei are Alistipes putredinis (JKB232A), Bacteroides cellulosilyticus (JKB161), Bacteroides intestinalis (JKB231), and Bacteroides dorei (JKB233), respectively.

11. The ameliorating agent according to any one of the above 6 to 10, in which the inflammatory disease, the autoimmune disease, or the infectious disease is at least one selected from the group consisting of an inflammatory bowel disease, an allergic disease, an infectious disease, diabetes, multiple sclerosis, rheumatoid arthritis, and a graft-versus-host rejection response.

12. A pharmaceutical product containing the ameliorating agent according to any one of the above 6 to 11.

13. A food containing the ameliorating agent according to any one of the above 6 to 11.

14. A microorganism that is Veillonella sp. (JKB232V).

15. An method for ameliorating an inflammatory disease, an autoimmune disease, or an infectious disease, the method comprising applying an intestinal bacterium belonging to the genus Veillonella and having an inflammation reducing activity or a substance derived from the intestinal bacterium and having an inflammation reducing activity, and the intestinal bacterium or the substance having at least one activity selected from an anti-inflammatory activity, an immunoregulation activity, an epithelial barrier restoring activity, an IL-10-inducing activity, and an IL-22-inducing activity.

16. Use of an intestinal bacterium belonging to the genus Veillonella and having an inflammation reducing activity or a substance derived from the intestinal bacterium and having an inflammation reducing activity for the manufacture of an ameliorating agent for an inflammatory disease, an autoimmune disease, and an infectious disease which has at least one activity selected from an anti-inflammatory activity, an immunoregulation activity, an epithelial barrier restoring activity, an IL-10-inducing activity, and an IL-22-inducing activity.

17. An intestinal bacterium belonging to the genus Veillonella and having an inflammation reducing activity or a substance derived from the intestinal bacterium and having an inflammation reducing activity for use as an ameliorating agent for an inflammatory disease, an autoimmune disease, and an infectious disease which has at least one activity selected from an anti-inflammatory activity, an immunoregulation activity, an epithelial barrier restoring activity, an IL-10-inducing activity, and an IL-22-inducing activity.

ADVANTAGEOUS EFFECTS OF INVENTION

A composition and an ameliorating agent for an inflammatory disease, an autoimmune disease, or an infectious disease according to the present invention have advantages of being effective for prevention, amelioration, or treatment of inflammatory diseases, autoimmune diseases, or infectious diseases and having a low risk of side effects because the composition and the ameliorating agent contain an intestinal bacterium capable of exhibiting an excellent inflammation reducing activity, or a substance derived from the intestinal bacterium and having an inflammation reducing activity.

BRIEF DESCRIPTION OF DRAWINGS

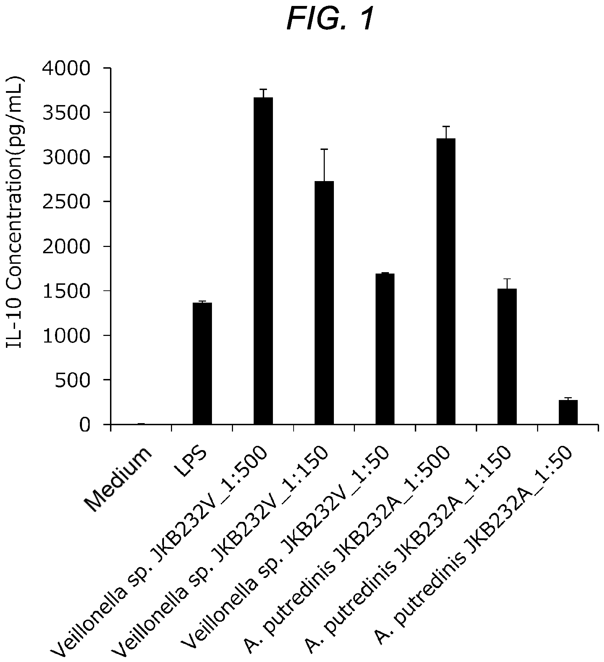

[FIG. 1] FIG. 1 is a diagram showing results of evaluating IL-10-inducing activities of Veillonella sp. (JKB232V) and Alistipes putredinis (JKB232A) isolated when each of Veillonella sp. (JKB232V) and Alistipes putredinis (JKB232A) isolated is co-cultured with BMDCs such that a ratio of BMDCs to intestinal bacteria (ratio of cells:dead bacteria cells) was 1:500, 1:150, or 1:50.

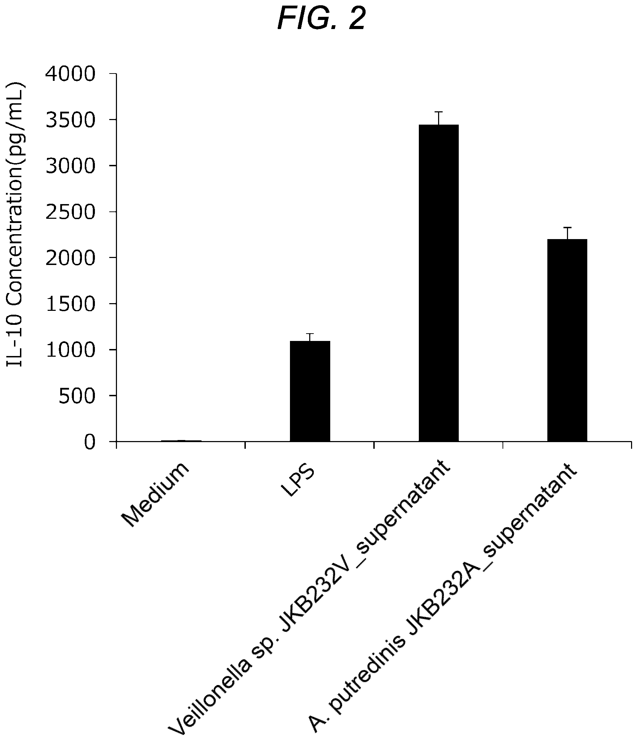

[FIG. 2] FIG. 2 is a diagram showing results of evaluating IL-10-inducing activities of Veillonella sp. (JKB232V) and Alistipes putredinis (JKB232A) isolated when a culture solution supernatant of each of Veillonella sp. (JKB232V) and Alistipes putredinis (JKB232A) isolated is co-cultured with BMDCs.

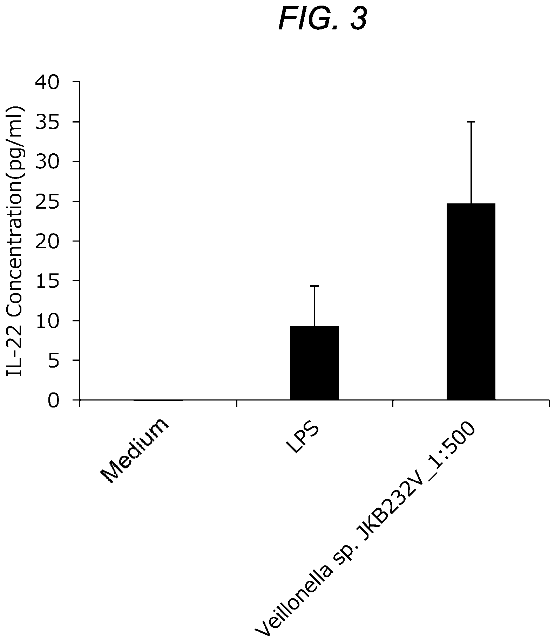

[FIG. 3] FIG. 3 is a diagram showing results of evaluating an IL-22-inducing activity of isolated Veillonella sp. (JKB232V) when isolated Veillonella sp. (JKB232V) is co-cultured with BMDCs such that a ratio of BMDCs to intestinal bacteria (ratio of cells:dead bacteria cells) was 1:500.

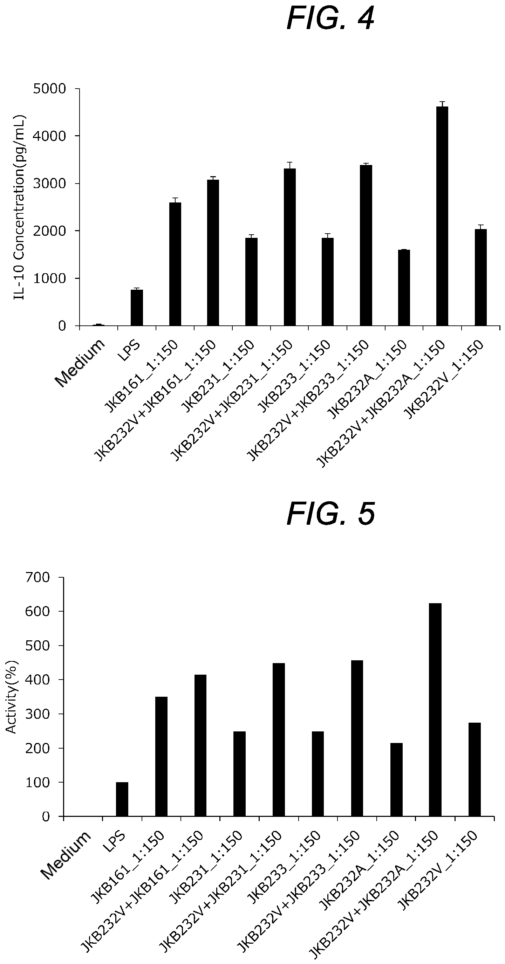

[FIG. 4] FIG. 4 is a diagram showing results of evaluating IL-10-inducing activities when dead bacteria cells obtained by subjecting Veillonella sp. (JKB232V) and an intestinal bacterium exhibiting an IL-10-inducing activity and belonging to Bacteroidetes to mixed culture at a ratio of approximately 1:1 are co-cultured with BMDCs.

[FIG. 5] FIG. 5 is a diagram showing results of evaluating an IL-10-inducing activities when dead bacteria cells obtained by subjecting Veillonella sp. (JKB232V) and an intestinal bacterium exhibiting an IL-10-inducing activity and belonging to Bacteroidetes to mixed culture at a ratio of approximately 1:1 are co-cultured with BMDCs.

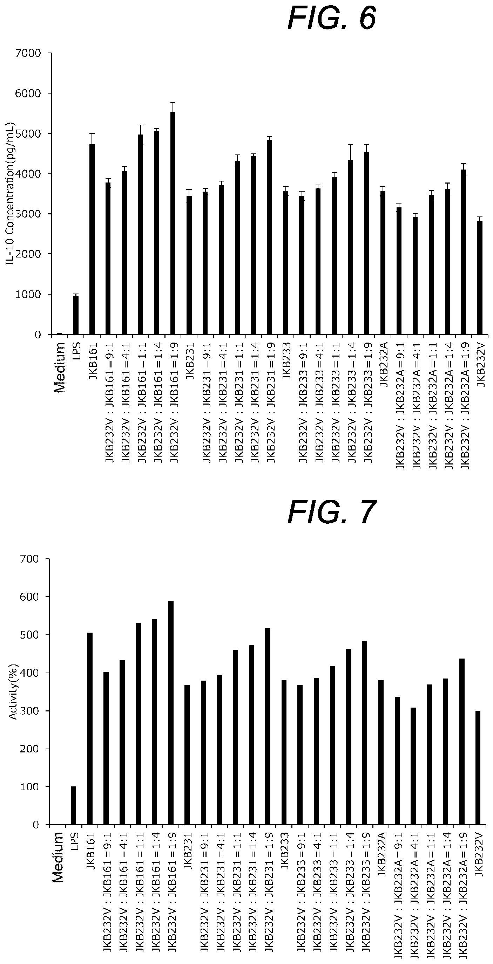

[FIG. 6] FIG. 6 is a diagram showing results of evaluating IL-10-inducing activities when dead bacteria cells of Veillonella sp. (JKB232V) and an intestinal bacterium exhibiting an IL-10-inducing activity and belonging to Bacteroidetes are mixed at a ratio of 9:1, 4:1, 1:1, 1:4, or 1:9, and are co-cultured with BMDCs.

[FIG. 7] FIG. 7 is a diagram showing values of IL-10-inducing activities when dead bacteria cells of Veillonella sp. (JKB232V) and an intestinal bacterium exhibiting an IL-10-inducing activity and belonging to Bacteroidetes are mixed at a ratio of 9:1, 4:1, 1:1, 1:4, or 1:9, and are co-cultured with BMDCs.

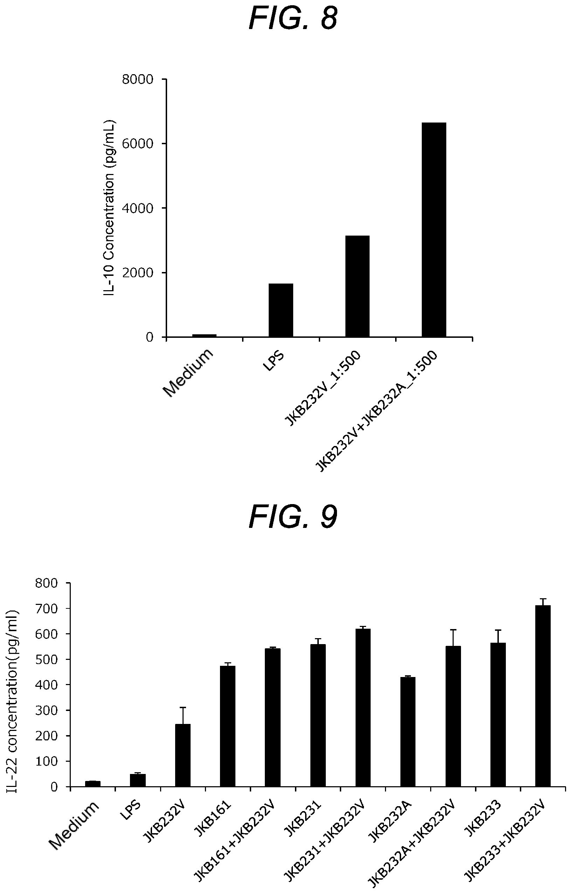

[FIG. 8] FIG. 8 is a diagram showing results of evaluating IL-10-inducing activities when Veillonella sp. (JKB232V) is co-cultured with human peripheral blood-derived dendritic cells (DCs) such that a ratio of human peripheral blood-derived dendritic cells (DCs) to an intestinal bacterium (a ratio of cells:dead bacteria cells) is 1:500 and when mixed bacteria cells obtained by mixing Alistipes putredinis (JKB232A) and Veillonella sp. (JKB232V) at a ratio of approximately 1:1 are co-cultured with human peripheral blood-derived dendritic cells (DCs) such that a ratio of human peripheral blood-derived dendritic cells (DCs) to an intestinal bacterium (a ratio of cells:dead bacteria cells) is 1:500.

[FIG. 9] FIG. 9 is a diagram showing results of evaluating an IL-22-inducing activity when each of Veillonella sp. (JKB232V) and mixed bacteria cells of Veillonella sp. (JKB232V) and an intestinal bacterium belonging to Bacteroidetes is co-cultured with BMDCs such that a ratio of BMDCs to an intestinal bacterium (a ratio of cells:dead bacteria cells or mixed dead bacteria cells) is 1:500.

DESCRIPTION OF EMBODIMENTS

In the present specification, "inflammation reduction" is a concept including any of reduction of inflammation, alleviation of inflammation, and amelioration of inflammation, and the mode of inflammation reduction is not particularly limited. The inflammation reducing activity can be evaluated by an activity of inducing IL-10 or IL-22 which is an anti-inflammatory cytokine, as described later in Examples.

The IL-10-inducing activity can be measured using, for example, a commercially available IL-10 measurement kit. Examples of the commercially available IL-10 measurement kit include Mouse IL-10 Duoset ELISA (manufactured by R&D), IL-10 (mouse) AlphaLISA Detection Kit (manufactured by PerkinElmer), and Human IL-10 AlphaLISA Detection Kit (PerkinElmer).

The IL-22-inducing activity can be measured using, for example, a commercially available IL-22 measurement kit. Examples of the commercially available IL-22 measurement kit include Mouse/Rat IL-22 Quantikine ELISA Kit (manufactured by R&D Systems).

When the inflammation reducing activity in the present specification is evaluated by the IL-10-inducing activity, the IL-10-inducing activity can be calculated from the following formula 1, assuming that a concentration of IL-10 produced from mouse-derived BMDCs when stimulation is performed with 1000 ng/ml of LPS is 100%. In the formula 1, the S value represents a concentration of IL-10 produced when stimulation is performed with an evaluation sample, the M value represents a concentration of IL-10 produced when stimulation is performed with a medium, and the L value represents a concentration of IL-10 produced when stimulation is performed with LPS.

$$\text{(Formula 1)} \quad (\text{S value} - \text{M value}) \div (\text{L value} - \text{M value}) \times 100 = \text{Activity value (\%)}$$

Regarding the IL-10-inducing activity in the present invention, the value calculated by the above formula 1 is preferably 100% or more, more preferably 200% or more, and still more preferably 400% or more when co-culture is performed, for example, in accordance with the method of Example 2 described later such that the ratio of BMDCs to intestinal bacteria (ratio of cells:dead bacteria cells) is 1:500.

Regarding the IL-22-inducing activity in the present invention, the value calculated by the above formula 1 is preferably 100% or more, more preferably 200% or more, and still more preferably 400% or more when co-culture is performed, for example, in accordance with the method of Example 2 described later such that the ratio of BMDCs to intestinal bacteria (ratio of cells:dead bacteria cells) is 1:500. When the IL-22-inducing activity is evaluated herein, the S value in the formula 1 is replaced by a value of a concentration of IL-22 produced when stimulation is performed with an evaluation sample, the M value in the formula 1 is replaced by a value of a concentration of IL-22 produced when stimulation is performed with a medium, and the L value in the formula 1 is replaced byo a value of a concentration of IL-22 produced when stimulation is performed with LPS as shown respectively.

One aspect of the present invention relates to a composition containing an intestinal bacterium having an inflammation reducing activity (hereinafter, also referred to as the composition according to the present invention). One kind of the intestinal bacterium having an inflammation reducing activity may be used alone, or two or more kinds thereof may be used in combination.

Examples of the intestinal bacterium having an inflammation reducing activity include intestinal bacteria belonging to the genus Veillonella. Examples of the intestinal bacteria belonging to the genus Veillonella include Veillonella tobetsuensis, Veillonella magna, Veillonella criceti, Veillonella ratti, Veillonella montpellierensis, Veillonella caviae, Veillonella dispar, Veillonella parvula, Veillonella rogosae, Veillonella atypica, Veillonella denticariosi, and Veillonella rodentium.

Among the intestinal bacteria, Veillonella sp. (JKB232V) is particularly preferred. Veillonella sp. (JKB232V) has been internationally deposited in NITE Patent Microorganisms Depositary, National Institute of Technology and Evaluation (postal code: 292-0818, #122, 2-5-8 Kazusakamatari, Kisarazu-shi, Japan) (Accession Number: NITE BP-03297) on September 30, 2020 by the present inventors.

The composition according to the present invention further contains an intestinal bacterium belonging to Bacteroidetes and having an inflammation reducing activity, or a substance derived from the intestinal bacterium and having an inflammation reducing activity. At least one or one or more of the intestinal bacteria belonging to Bacteroidetes and having an inflammation reducing activity or the substances derived from the intestinal bacteria and having an inflammation reducing activity may be used in combination. The inflammation reducing effect can be further improved by further containing, in addition to the intestinal bacterium belonging to the genus Veillonella or the substance derived from the intestinal bacterium and having an inflammation reducing activity, the intestinal bacterium belonging to Bacteroidetes and having an inflammation reducing activity or the substance derived from the intestinal bacterium and having an inflammation reducing activity.

Examples of the intestinal bacterium belonging to Bacteroidetes and having an inflammation reducing activity include an intestinal bacterium belonging to the genus Alistipes, an intestinal bacterium belonging to the genus Bacteroides, an intestinal bacterium belonging to the genus Parabacteroides, and an intestinal bacterium belonging to the genus Butyricimonas. From the viewpoint of exhibiting an excellent inflammation reducing activity, an intestinal bacterium belonging to the genus Alistipes or an intestinal bacterium belonging to the genus Bacteroides is preferred among them.

Examples of the intestinal bacterium belonging to the genus Alistipes include Alistipes putredinis and Alistipes shahii. Alistipes may be abbreviated as A. Examples of the intestinal bacterium belonging to the genus Bacteroides include Bacteroides caccae, Bacteroides cellulosilyticus, Bacteroides dorei, Bacteroides eggerthii, Bacteroides finegoldii, Bacteroides fragilis, Bacteroides intestinalis, Bacteroides koreensis, Bacteroides massiliensis, Bacteroides ovatus, Bacteroides salyersiae, Bacteroides stercoris, Bacteroides thetaiotaomicron, Bacteroides uniformis, Bacteroides vulgatus, and Bacteroides xylanisolvens. Examples of the intestinal bacterium belonging to the genus Parabacteroides include Parabacteroides distasonis, Parabacteroides goldsteinii, Parabacteroides gordonii, Parabacteroides johnsonii, and Parabacteroides merdae. Examples of the intestinal bacterium belonging to the genus Butyricimonas include Butyricimonas faecihominis.

Among them, from the viewpoint of being excellent in the inflammation reducing activity, Alistipes putredinis, Bacteroides cellulosilyticus, Bacteroides intestinalis, Bacteroides dorei are more preferred.

Among the intestinal bacteria, Alistipes putredinis (JKB232A), Bacteroides cellulosilyticus (JKB161), Bacteroides intestinalis (JKB231), and Bacteroides dorei (JKB233) are particularly preferred.

Alistipes putredinis (JKB232A) has been internationally deposited in NITE Patent Microorganisms Depositary, National Institute of Technology and Evaluation (postal code: 292-0818, #122, 2-5-8 Kazusakamatari, Kisarazu-shi, Japan) (Accession Number: NITE BP-03326) on November 27, 2020 by the present inventors.

Bacteroides cellulosilyticus (JKB161) has been internationally deposited in NITE Patent Microorganisms Depositary, National Institute of Technology and Evaluation (postal code: 292-0818, #122, 2-5-8 Kazusakamatari, Kisarazu-shi, Japan) (Accession Number: NITE BP-03063) on November 20, 2019 by the present inventors.

Bacteroides intestinalis (JKB231) has been internationally deposited in NITE Patent Microorganisms Depositary, National Institute of Technology and Evaluation (postal code: 292-0818, #122, 2-5-8 Kazusakamatari, Kisarazu-shi, Japan) (accession number: NITE BP-03064) on November 20, 2019 by the present inventors.

Bacteroides dorei (JKB233) has been internationally deposited in NITE Patent Microorganisms Depositary, National Institute of Technology and Evaluation (postal code: 292-0818, #122, 2-5-8 Kazusakamatari, Kisarazu-shi, Japan) (Accession Number: NITE BP-03066) on November 20, 2019 by the present inventors.

Examples of the intestinal bacterium used in one aspect of the present invention include bacteria as derived strains of Veillonella sp. (JKB232V), Alistipes putredinis (JKB232A), Bacteroides cellulosilyticus (JKB161), Bacteroides intestinalis (JKB231), or Bacteroides dorei (JKB233).

The bacteria as derived strains are strains derived from descendants or original species, and include strains whose biological activity is at least equivalent to that of a bacterium as a derivation source and which is modified (for example, genetically modified). That is, the bacteria as derived strains having an inflammation reducing activity that is at least equivalent to that of Veillonella sp. (JKB232V), Alistipes putredinis (JKB232A), Bacteroides cellulosilyticus (JKB161), Bacteroides intestinalis (JKB231), or Bacteroides dorei (JKB233) are exemplified.

Examples of the bacteria as derived strains include bacteria having a 16s rRNA sequence having an identity of at least 98.7%, 99%, 99.5%, or 99.9% with a 16s rRNA sequence of Veillonella sp. (JKB232V), Alistipes putredinis (JKB232A), Bacteroides cellulosilyticus (JKB161), Bacteroides intestinalis (JKB231), or Bacteroides dorei (JKB233).

Among the derived strains, a strain suitable for the present invention may be identified by other nucleotide sequences of Veillonella sp. (JKB232V), Alistipes putredinis (JKB232A), Bacteroides cellulosilyticus (JKB161), Bacteroides intestinalis (JKB231), or Bacteroides dorei (JKB233).

Examples of the sequence to be used for identification of a derived strain of Alistipes putredinis (JKB232A), Bacteroides cellulosilyticus (JKB161), Bacteroides intestinalis (JKB231), or Bacteroides dorei (JKB233) include an hsp60 sequence. It is preferable that the derived strains respectively have sequence identities of at least 98%, 99%, 99.5%, and 99.9% with sequences corresponding to Alistipes putredinis (JKB232A), Bacteroides cellulosilyticus (JKB161), Bacteroides intestinalis (JKB231), and Bacteroides dorei (JKB233).

Examples of the sequence used for identification of a derived strain of Veillonella sp. (JKB232V) include a rpoB sequence and a dnaK sequence. The derived strain preferably has a sequence having a sequence identity of at least 98%, 99%, 99.5%, or 99.9% with a sequence corresponding to Veillonella sp. (JKB232V).

In DNA-DNA hybridization with each corresponding derivation source strain, the derived strains preferably have an identity of at least 70%, 75%, 80%, 85%, 90%, 95%, 98%, or 99% (Wayne et al., Int. J. Syst. Bacteriol., 1987, 37:463-464).

The derived strains preferably have a sequence having a sequence identity of at least 95%, 96%, 97%, 98%, 99%, 99.5%, or 99.9% in average nucleotide identity (ANI) which is an average value of homology values in orthologous regions on two genomic sequences (Goris, J. et al. Int. J. Syst. Evol. Microbiol., 2007, 57:81-91. Richter, M. & Rossello-Mora, R. Proc. Natl. Acad. Sci. USA, 2009, 106:19126-19131.).

Among the derived strains, a strain suitable for the present invention can be identified by the use of a deposit of Veillonella sp. (JKB232V), Alistipes putredinis (JKB232A), Bacteroides cellulosilyticus (JKB161), Bacteroides intestinalis (JKB231), or Bacteroides dorei (JKB233), an analysis of a restriction fragment derived from the deposit, and/or a PCR analysis, for example, by the use of a fluorescent amplified fragment length polymorphism (FAFLP) and a repetitive DNA element (rep)-PCR fingerprinting, or a protein profiling, or partial 16S or 23s rDNA sequencing.

Among the derived strains, examples of preferred strains for use in the present invention include strains having the same pattern as the pattern of Veillonella sp. (JKB232V), Alistipes putredinis (JKB232A), Bacteroides cellulosilyticus (JKB161), Bacteroides intestinalis (JKB231), or Bacteroides dorei (JKB233) when analysis is performed by amplified ribosomal DNA restriction analysis (ARDRA) using a Sau3AI restriction enzyme [Srutkova et al. J. Microbiol.Methods, 2011, 87(1):10-6].

Among the derived strains, examples of the strain suitable for the present invention include strains that can be identified as strains having the same carbohydrate fermentation pattern as that of Veillonella sp. (JKB232V), Alistipes putredinis (JKB232A), Bacteroides cellulosilyticus(JKB161), Bacteroides intestinalis (JKB231), or Bacteroides dorei (JKB233).

Among the derived strains, the strain suitable for the present invention may be identified using any appropriate method. For example, the bacterial strains having proliferation patterns, types of metabolism, and/or surface antigens similar to those of Veillonella sp. (JKB232V), Alistipes putredinis (JKB232A), Bacteroides cellulosilyticus (JKB161), Bacteroides intestinalis (JKB231), or Bacteroides dorei (JKB233) are useful in the present invention.

Among the derived strains, the strain suitable for the present invention preferably has an inflammation reducing activity equivalent to that of Veillonella sp. (JKB232V), Alistipes putredinis (JKB232A), Bacteroides cellulosilyticus (JKB161), Bacteroides intestinalis (JKB231), or Bacteroides dorei (JKB233). The inflammation reducing activity can be evaluated, for example, by an IL-10-inducing activity, an IL-22-inducing activity, and the like, which are described later in Examples.

The composition according to the present invention preferably contains, regarding the intestinal bacterium having an inflammation reducing activity, a microorganism group in an amount of $10^5$ microorganisms/g to $10^{12}$ microorganisms/g, or functional ingredients derived from the microorganism group. In the composition and the ameliorating agent for an inflammatory disease, an autoimmune disease, or an infectious disease, it is preferable to use an intestinal bacterium having an inflammation reducing activity by artificial culture and growth of the intestinal bacterium. The composition and shape of the intestinal bacterium according to the present invention are appropriately devised in view of the application of the inflammation reducing activity thereof, and the production method of the intestinal bacterium is not particularly limited. Normally, the intestinal bacterium may be cultured in a culture medium suitable for the growth of the intestinal bacterium under the control that does not cause contamination of foreign substances that hinder the culture.

As the intestinal bacterium used in the present invention, one obtained by concentrating and preserving an artificially cultured and proliferated intestinal bacterium while keeping the intestinal bacterium alive may be used. The method for concentrating and preserving the intestinal bacterium may be in any form as long as the intestinal bacterium is living up to the time of use, and the degree of concentration and purification may be any degree as long as medium ingredients that can cause an allergy are sufficiently removed. Examples of the concentrating and preserving method include a method in which concentrating by centrifugation is performed, and the concentrated product is further dried to prepare a dry powder, or is solidified during drying to obtain a form such as a solid form. The normal means generally applied may be freely selected.

As the intestinal bacterium used in the present invention, one obtained by killing an artificially cultured and proliferated intestinal bacterium by a heat treatment or the like may be used.

As one aspect of the present invention, there is provided an ameliorating agent for an inflammatory disease, an autoimmune disease, or an infectious disease (hereinafter, also abbreviated as an agent according to the present invention), which contains an intestinal bacterium having an inflammation reducing activity or a substance derived from the intestinal bacterium and having an inflammation reducing activity and which has at least one activity selected from an anti-inflammatory activity, an immunoregulation activity, an epithelial barrier restoring activity, an IL-10-inducing activity, and an IL-22-inducing activity. The intestinal bacterium used in the present invention has an excellent IL-10-inducing activity or IL-22-inducing activity, and has an activity such as an enteritis reducing activity. Therefore, the agent according to the present invention can effectively ameliorate an inflammatory disease, an autoimmune disease, or an infectious disease.

Examples of organs and cells affected by the inflammatory disease, the autoimmune disease, or the infectious disease include a digestive tract, some organs of the digestive tract, a liver, liver cells, a pancreas, pancreatic cells, a kidney, kidney cells, a blood vessel, a respiratory organ, a cerebrospinal cord, blood cells, an eye, a joint, a muscle, skin, immune cells, epithelial cells, epidermal cells, nerve cells, endothelial cells, and fibroblasts.

Examples of some organs of the digestive tract include an esophagus, a stomach, and an intestine [for example, a small intestine (such as a duodenum, a jejunum, and an ileum) and/or a large intestine (such as a cecum, an ascending colon, a transverse colon, a descending colon, and a sigmoid colon)].

Examples of the epithelial cells include intestinal epithelial cells. Examples of the immune cells include dendritic cells, monocytes/macrophages, T cells, and neutrophils.

Examples of the inflammatory disease, the autoimmune disease, or the infectious disease include an inflammatory bowel disease (IBD), ulcerative colitis, Crohn's disease, irritable bowel syndromes, sprue, autoimmune arthritis, rheumatoid arthritis, type 1 diabetes, multiple sclerosis, a graft-versus-host rejection response associated with bone marrow transplantation, osteoarthritis, juvenile chronic arthritis, Lyme arthritis, psoriatic arthritis, reactive arthritis, spondylarthrosis, systemic lupus erythematosus, insulin-dependent diabetes mellitus, thyroiditis, asthma, psoriasis, dermatitis scleroderma, atopic dermatitis, a graft-versus-host rejection response, acute or chronic immune diseases related to organ transplantation, sarcoidosis, atherosclerosis, disseminated intracapillary coagulation, Kawasaki disease, Graves' disease (Basedow-Krankheit), nephrotic syndromes, chronic fatigue syndromes, Wegener's granulomatosis, Henoch-Schonlein purpura, microscopic vasculitis in kidneys, chronic active hepatitis, uveitis, septic shock, toxicogenic shock syndromes, sepsis syndromes, cachexia, AIDS (acquired immunodeficiency syndrome), transverse myelitis, Huntington's chorea, Parkinson's disease, Alzheimer's disease, cerebral apoplexy, primary biliary cirrhosis, hemolytic anemia, polyglandular deficiency syndromes type 1, polyglandular deficiency syndromes type 2, Schmidt's syndromes, adult respiratory distress syndromes, alopecia, alopecia areata, seronegative arthrosis, arthrosis, Reiter's disease, psoriatic arthrosis, Chlamydia infection, arthrosis related to Yersinia and salmonella infection, spondylarthrosis/arteriosclerosis, allergic diseases (for example, atopic allergies, food allergies, pollen allergies, allergic rhinitis, anaphylaxis, pet allergies, latex allergies, drug allergies, allergic rhinitis and conjunctivitis), autoimmune blister diseases, pemphigus vulgaris, pemphigus foliaceus, bullous pemphigoid, Linear IgA diseases, autoimmune hemolytic anemia, Coombs test positive hemolytic anemia, acquired pernicious anemia, juvenile pernicious anemia, myalgic encephalomyelitis/Royal Free disease, chronic mucocutaneous candidiasis, giant cell arteritis, primary sclerosing hepatitis, cryptogenic autoimmune hepatitis, acquired immunodeficiency related diseases, Hepatitis C, common variable immunodeficiency (cryptogenic hypogammaglobulinemia), dilated cardiomyopathy, fibrotic lung diseases, cryptogenic fibrosing alveolitis, interstitial pneumonia after inflammation, interstitial pneumonia, tissue diseases associated interstitial lung diseases, mixed connective tissue diseases associated lung diseases, systemic sclerosis associated interstitial lung diseases, rheumatoid arthritis associated interstitial lung diseases, systemic lupus erythematosus associated lung diseases, dermatomyositis/polymyositis associated lung diseases, Sjogren syndromes associated lung diseases, ankylosing spondylitis associated lung diseases, vasculitic diffuse lung diseases, hemosiderosis lung diseases, drug-induced interstitial pulmonary diseases, radiation fibrosis, bronchiolitis obliterans, chronic eosinophilic pneumonia, lymphocyte infiltration lung diseases, interstitial pneumonia after infection, gouty arthritis, autoimmune hepatitis, type 1 autoimmune hepatitis (classic autoimmune or lupoid hepatitis), type 2 autoimmune hepatitis (anti-LKM1 antibody hepatitis), autoimmune hypoglycemia, insulin receptor abnormalism type B due to acanthosis nigricans, hyperthyroidism, immune diseases associated with organ transplantation, chronic immune diseases associated with organ transplantation, osteoarthritis, primary sclerosing cholangitis, idiopathic leukopenia, autoimmune neutropenia, renal disease NOS, glomerulotubular nephritis, microscopic vasculitis in kidneys, discoid erythematosus, idiopathic male infertility or NOS, sperm autoimmunity, multiple sclerosis (related to all subtypes), insulin-dependent diabetes, sympathetic ophthalmia, tissue diseases due to pulmonary hypertension, good pasture syndromes, pulmonary symptoms of polyarteritis nodosa, acute rheumatic fever, rheumatoid spondylitis, Still's diseases, systemic sclerosis, Takayasu's diseases/ arteritis, autoimmune thrombocytopenia, idiopathic thrombocythemia, autoimmune thyropathy, hyperthyroidism, hypothyroidism (Hashimoto's diseases), atrophic autoinmiune hypothyroidism, primary myxedema, uveitis caused by crystalline lens, primary vasculitis, vitiligo, eosinophilic esophagitis, hypereosinophilic syndromes, eosinophilic gastroenteritis, and cutaneous lupus erythematosus.

The composition according to the present invention can also be used as a composition for preventing or treating an infectious disease of an individual whose resistance to the infectious disease is impaired by damage caused by excessive inflammation via immunity.

Examples of such an infectious pathogen that impair the maintenance or recovery of a homeostatic state (homeostasis) of a host and, as a result, causes immunopathic tissue damage include salmonellas, shigellas, Clostridium difficile (C. difficile), mycobacteria (tuberculosis in the case of a disease), protozoa (malaria in the case of a disease), thread-shaped threadworms (filariasis in the case of a disease), schistosomes (schistosomiasis in the case of a disease), toxoplasmas (toxoplasmosis in the case of a disease), leishmania (leishmaniasis in the case of a disease), HCV and HBC (hepatitis C and hepatitis B in the case of a disease), and herpes simplex viruses (herpes in the case of a disease).

The target of the present invention is preferably a mammal. Examples of mammals include omnivores (for example, humans, rats, and pigs), carnivores (for example, dogs and cats), and herbivores (for example, horses and rabbits). The target is preferably a human.

The composition or agent according to the present invention can be used to regulate inflammation of cells, tissues, or organs in a target. In the present invention, the term "regulation of inflammation" refers to diminishment, reduction, and/or inhibition of inflammation. In the present invention, the term "inflammation" refers to at least one selected from redness, swelling, pain, tenderness, heat, and loss of function in cells, tissues, or organs, which are caused in an inflammation process induced by an excess reaction of an immune system.

Specific examples of the inflammation include inflammation in a digestive tract, some organs of a digestive tract (for example, an intestine), a liver, liver cells, a pancreas, pancreas cells (for example, islets of Langerhans), a kidney, kidney cells, a blood vessel, a respiratory organ, a cerebrospinal cord, blood cells, an eye, a joint, a muscle, skin, epithelial cells, epidermal cells, nerve cells, endothelial cells, and fibroblasts.

In one aspect of the present invention, the number of cells in which inflammation is caused in the target after administration of the composition or agent according to the present invention is preferably smaller than the number of cells in which inflammation is caused in the target before the administration. For example, the number of cells is preferably reduced by at least 10%, 20%, 30%, 40%, or 50%.

In one aspect of the present invention, the amount of tissue or organ in which inflammation is caused in the target after administration of the composition or agent according to the present invention is preferably smaller than the amount of tissue or organ in which inflammation is caused in the target before administration. For example, the amount of tissue or organ is preferably reduced by at least 10%, 20%, 30%, 40%, or 50%.

In one aspect of the present invention, the composition or the agent according to the present invention preferably increases the production amount of IL-10 in the target. IL-10 acts on immune cells such as T cells or macrophages to directly inhibit the activation of the cells and weaken an antigen presenting ability of the macrophages, thereby settling the immune response. IL-10 is related to the development of ulcerative colitis (Franke A, et al., Nat Genet. 2008;40:1319-23).

In one aspect of the present invention, the composition or the agent according to the present invention preferably increases the production amount of IL-22 in the target. IL-22 is related to the barrier restoring action of epithelial cells.

In one aspect of the present invention, the composition or the agent according to the present invention is used to reduce the inflammation level and/or to ameliorate intestinal microbial flora in a digestive tract of a target or a part of the digestive tract. The digestive tract includes an esophagus, a stomach, and an intestine [for example, a small intestine (such as a duodenum, a jejunum, and an ileum) and/or a large intestine (such as a cecum, an ascending colon, a transverse colon, a descending colon, and a sigmoid colon)].

Specifically, the inflammation level after administration of the composition or agent according to the present invention is preferably reduced by, for example, at least 10%, 20%, 30%, 40%, or 50% as compared with the inflammation level in the digestive tract of the target before the administration.

In the present specification, the term "intestinal microbial flora" refers to microorganisms that live in a digestive tract of a host animal. These microorganisms have various metabolic functions, structural functions, protection functions, and other useful functions.

Specific examples of the amelioration of intestinal microbial flora include increasing the number and/or type of desired microorganisms present in the intestine of the target (for example, a host) and/or increasing the activity of the desired microorganisms in terms of the metabolic functions, structural functions, protection functions, and other useful functions. Further, examples of the amelioration of intestinal microbial flora include reducing the number and/or type of undesired microorganisms present in the intestine of the target (for example, a host) and/or reducing the activity of the undesired microorganisms in terms of the metabolic functions, structural functions, protection functions, and other useful functions.

Examples of forms of the desired microorganism in the intestine of the host include microorganisms having protection functions and useful functions. Examples of the desired microorganism in the intestine of the host include bacteria belonging to Firmicutes and Bacteroidetes.

Examples of the form of the undesired microorganism in the intestine of the host include microorganisms that can interfere with the metabolic functions, the structural functions, the protection functions, and other useful functions of the desired microorganisms having the protection functions and useful functions, and microorganisms that cause inflammation and/or diarrhea. Examples of the undesired microorganisms in the intestine of the host include Escherichia coli.

As the amelioration of the intestinal microbial flora, for example, it is preferable that administration of the composition or the agent according to the present invention to a target having an inflammatory bowel disease may cause a change in the balance of the microbial flora between a desired microorganism and an undesired microorganism in the intestine. Specifically, for example, it is preferable that the number of desired microorganisms present in the intestine of the target is increased by at least 10%, 20%, 30%, 40%, or 50%, or more than 100% by comparing a level in the target after administration of the composition or the agent according to the present invention with a level before administration.

The composition according to the present invention can be used as an active ingredient of an ameliorating agent for an inflammatory disease, an autoimmune disease, or an infectious disease [for example, pharmaceutical products, foods, health promotion agents, nutritional auxiliary agents (for example, supplements), and food additives]. The agent according to the present invention can be used as it is or used together with common ingredients to form an ameliorating agent for an inflammatory disease, an autoimmune disease, or an infectious disease, and can be applied to non-human animals and humans (for example, administration, ingestion, and inoculation).

The application form of the agent according to the present invention is not particularly limited, and the agent can be used in any application form such as rectal administration using a suppository or the like, or an administration using a colonoscope, in addition to oral administration and enteral administration. The agent according to the present invention can be applied to various products such as foods and drinks, pharmaceuticals, feeds, and pet foods because the agent can be used in any application form to exhibit an inflammation reducing activity. The agent according to the present invention may be ingested (administered) as a supplement or the like, or may be used as an additive for imparting an activity of ameliorating an inflammatory disease, an autoimmune disease, or an infectious disease to a composition such as foods and drinks.

In addition, the dosage forms of the agent according to the present invention may be any of a solid form, a semi-solid form, a liquid form, and the like, and is appropriately set according to the kind and application of the product. The agent according to the present invention may contain additives such as water, fats and oils, waxes, hydrocarbons, fatty acids, higher alcohols, esters, plant extracts, water-soluble polymers, surfactants, metallic soaps, alcohols, polyhydric alcohols, pH adjusters, antioxidants, ultraviolet inhibitors, preservatives, flavor or fragrances, powders, thickeners, pigments, and chelating agents, as long as the effects of the present invention are not impaired.

The agent according to the present invention may be blended with other anti-inflammatory ingredients in accordance with the form, application, and the like thereof, as long as the effects of the present invention are not impaired. Examples of such anti-inflammatory ingredients include vitamin C, squalane, niacin, niacinamide, long-chain hyaluronic acids, placenta extracts, sorbitol, chitin, chitosan, and various types of plant extracts. The blending amount of these is not limited as long as the effects of the present invention are not impaired.

When the agent according to the present invention is used as a food, the agent according to the present invention is provided as a food exhibiting desired effects by using the agent as it is or adjusting the agent to a desired form by combination with other food materials or additive ingredients. Such foods include, in addition to general foods and drinks, health foods, functional foods, nutritional supplements, and supplements, and examples of such foods include health function foods such as foods with a disease risk reduction indication (for example, specific insurance foods, nutritional functional foods, and foods with function claims) and foods for patients.

Specific examples of forms of the foods include, but are not particularly limited to, liquid foods such as drinks, soups, non-alcoholic drinks, alcoholic drinks, jelly drinks, and functional drinks; semi-solid foods such as jelly and yogurt; fermented foods such as miso and fermented drinks; various sweets, for example, western style confections such as cookies and cakes, Japanese style confections such as bean-jam bun and sweet bean jelly, candies, gums, gummi, cold confections, and frozen confections; products containing oil such as edible oil, dressing, mayonnaise, and margarine; carbohydrate-containing foods such as rice, rice cakes, noodles, breads, and pastas; processed livestock foods such as hams and sausages; processed sea foods such as kamaboko, dried fishes, and salted fishes; processed vegetable foods such as pickles; boil-in-the-bag foods such as curries, starchy sauces, and Chinese soups; instant foods such as instant soups and instant miso soups; microwaveable foods; processed products using eggs, and processed products of seafoods or livestock meat; and seasonings. Healthy foods prepared in the form of powder, granules, tablets, capsules, liquid, paste, or jelly may also be used. These foods can be used for the applications described above. The food for patients is provided for patients requiring prevention, amelioration, or treatment of inflammatory diseases, autoimmune diseases, or infectious diseases.

When the agent according to the present invention is used for foods, the blending amount of the intestinal bacterium having an inflammation reducing activity to the foods varies depending on the form of foods and drinks. Those skilled in the art can appropriately set the amount while predicting the effect based on known information, an animal test, or the like according to the used intestinal bacterium having an inflammation reducing activity. Examples of the blending amount of the intestinal bacterium having an inflammation reducing activity which can be set in this manner include a range serving as the amount of a microorganism group of preferably $10^5$ microorganisms/g to $10^{12}$ microorganisms/g, and more preferably $10^6$ microorganisms/g to $10^{11}$ microorganisms/g or the amount of functional ingredients derived from the microorganism group.

Further, when the agent according to the present invention is used for foods, the agent according to the present invention can also be provided alone or in combination with other ingredients as a food additive for ameliorating an inflammatory disease, an autoimmune disease, or an infectious disease. When the agent according to the present invention is used as a food additive, the content of the intestinal bacterium having an inflammation reducing activity in the food additive, the addition amount of the food additive to foods and drinks, and the like may be appropriately set so that the content described above can be satisfied by the intestinal bacterium having an inflammation reducing activity in the foods and drinks as an addition target.

The composition of the present invention can be formulated by a known formulation method. The composition of the present invention can be used orally or parenterally as, for example, a capsule, a tablet, a pill, a liquid, powder, a granule, a fine grain, a film coating agent, a pellet, a troche, a sublingual agent, a peptizing agent, a buccal agent, a paste, a syrup agent, a suspension, an elixir agent, an emulsion, a coating agent, an ointment agent, a plaster, a cataplasm, a percutaneous absorption type formulation, a lotion, a suction agent, an aerosol, an injection, or a suppository.

In the formulation, the composition according to the present invention can be appropriately combined with a carrier which is acceptable in terms of pharmacology or foods and drinks, specifically, sterilized water, physiological saline, vegetable oil, solvents, basic agents, emulsifying agents, suspension agents, surfactants, stabilizers, flavoring agents, perfuming agents, excipients, vehicles, preservatives, binders, diluents, isotonizing agents, soothing agents, extenders, disintegrating agents, buffers, coating agents, lubricants, coloring agents, sweeteners, thickening agents, flavoring and odor improving agents, solubilizing agents, or other additives.

In the formulation, particularly in a formulation intended for oral administration, it is preferable to combine the composition according to the present invention with a composition that enables efficient delivery of the composition to the large intestine.

The composition or method that enables delivery to the large intestine is not particularly limited, and a known composition or method can be appropriately adopted. For example, a pH sensitive composition, more specifically, an enteric polymer that releases an inclusion when pH becomes alkaline after passing through the stomach is exemplified. When the pH sensitive composition is used for the formulation, the pH sensitive composition is preferably a polymer having a pH of 6.8 to 7.5 as a threshold value at which the composition is decomposed. Such a numerical range is a range of pH shift to the alkaline side occurring in a far part of the stomach, so that the numerical range is a range suitable for use in delivery to the large intestine.

Examples of the composition that enables delivery to the large intestine include a composition that ensures delivery to the large intestine by delaying the release of the inclusion for about 3 hours to 5 hours approximately equivalent to the small intestine passing time. In the formulation of the composition that delays the release, formulation is exemplified in which a hydrogel is used as a shell and the hydrogel is hydrated and expanded by being contact with a gastrointestinal fluid and thus effectively releases an inclusion. An example of a metering unit for delaying the release is a drug-containing composition in which a drug is coated or a selective coating material is contained.

Examples of the selective coating material include a biodegradable polymer, a polymer that is gradually hydrolyzed, a polymer that gradually dissolves, and/or an enzymatic degradable polymer. The coating material suitable for efficiently delaying the release is not particularly limited, and examples thereof include a cellulose-based polymer such as hydroxypropyl cellulose, an acrylic acid polymer and an acrylic acid copolymer such as a methacrylic acid polymer and a methacrylic acid copolymer, and a vinyl acid polymer and a vinyl acid copolymer such as polyvinylpyrrolidone.

Examples of the composition that enables delivery to the large intestine include a bioadhesive composition (for example, a polymer described in US6,368,586) that specifically adheres to a large intestine mucosa, and a composition in which a protease inhibitor is incorporated in order to particularly protect a biological formulation from degradation caused by a protein degradation enzyme activity in a digestive tract.

Examples of a system that enables delivery to the large intestine include delivery systems to the large intestine which cause release by a pressure change, such as a delivery system in which a content is released by utilizing a pressure change in the far part of the stomach generated as a result of gas production by bacterial fermentation. The system is not particularly limited, and more specifically, a capsule formed of an inclusion which is coated with a hydrophobic polymer (for example, ethyl cellulose) and dispersed in a basic agent of a suppository is exemplified.

Examples of the system that enables delivery to the large intestine include a delivery system to the large intestine in which specific decomposing is caused by an enzyme (for example, a carbohydrate hydrolase, and a carbohydrate reductase) present in the large intestine is exemplified. The system is not particularly limited, and more specifically, systems using food ingredients such as non-starch polysaccharides, amylose, xanthan gum, and azopolymers are exemplified.

When the agent according to the present invention is used for pharmaceutical products, a blending ratio of the intestinal bacterium having an inflammation reducing activity to the pharmaceutical products varies depending on the form of the pharmaceutical products or the like. For example, in the case of an oral administration formulation or an injection, a range serving as the amount of a microorganism group of preferably $10^5$ microorganisms/g to $10^{12}$ microorganisms/g, and more preferably $10^6$ microorganisms/g to $10^{11}$ microorganisms/g or the amount of functional ingredients derived from the microorganism group is exemplified.

An application amount according to the present invention (for example, administration, ingestion, and inoculation) is not particularly limited as long as it is an effective dose. Normally, the application amount is preferably $10^5$ microorganisms/g to $10^{12}$ microorganisms/g, and more preferably $10^6$ microorganisms/g to $10^{11}$ microorganisms/g per day in general, in terms of the number of intestinal bacteria having an inflammation reducing activity as an active ingredient. The application amount is preferably one or more times a day (for example, one to three times a day), and can be appropriately increased or decreased depending on the age, pathological conditions, and symptoms.

Those skilled in the art can easily determine an appropriate dose of intestinal bacteria having an inflammation reducing activity, which are to be administered to a target, without excessive experiments. Normally, a doctor determines a dose for suitable respective patients, and the dose depends on various factors including the activity of a strain to be used, the metabolic stability and action length of the strain, the age, the body weight, the overall health, the sex, the meal, the administration method and the number of administration times, the excretion rate, the combination of drugs, the severity in a specific state, and the treatment received by an individual. The dose disclosed in the present specification is an example of an average case. Of course, there are individual aspects in which the ranges of higher or lower doses are suitable, and such aspects are also included in the present invention.

When the composition according to the present invention is used as a pharmaceutical composition, the composition may be used in combination with a known pharmaceutical composition used for inhibition of immunization or inflammation. Examples of the known pharmaceutical composition include, but are not particularly limited to, corticosteroid, mesalazine, mesalamine, sulfasalazine, sulphasalazine derivatives, immunosuppressants, cyclosporin A, mercaptopurine, azathioprine, prednisone, methotrexate, antihistamine, glucocorticoid, epinephrine, theophylline, sodium cromoglicate, antileukotriene, anticholinergic agents for rhinitis, anticholinergic decongestant, mast cell stabilizing agents, a monoclonal anti-IgE antibody, a TNF-α inhibitor, an anti-IL-1 antibody, an anti-IL-6 antibody or an anti-IL-6 receptor antibody, an anti-IL-12 antibody, an anti-IL-15 antibody, an anti-IL-17 antibody or an anti-IL-17 receptor antibody, an anti-IL-23 antibody, a T-cell-selective co-stimulation regulator, an anti-α4β7 integrin antibody, an anti-GM-CSF antibody or anti-GM-CSF receptor antibody, anti-BLyS antibody, a JAK inhibitor, vaccines (preferably, vaccines used in vaccine inoculation in which the amount of allergen is gradually increased), and at least one therapeutic composition selected from the group consisting of these combinations. These pharmaceutical compositions are preferably used in combination with the composition according to the present invention.

Examples

Examples are shown below, but the present invention is not limited to the following Examples.

[Example 1] Isolation of Intestinal Bacterium

In the case of fecal microbiota transplantation for a patient with ulcerative colitis, it was confirmed that the intestinal bacteria present in an intestine of a donor colonized an intestine of a patient with confirmed remission after fecal microbiota transplantation. Then, the isolation of the intestinal bacteria expected to be effective for ulcerative colitis was attempted from donor feces samples, and Veillonella sp., Alistipes putredinis, Bacteroides cellulosilyticus, Bacteroides intestinalis, and Bacteroides dorei were successfully isolated.

[Example 2] Evaluation of Cytokine-IL-10-inducing Activity of Isolated Intestinal Bacteria

The fecal microbiota transplantation into patients with inflammatory bowel diseases was recognized to ameliorate symptoms, so that it was assumed that some of the anti-inflammatory activities act. Bacteroides fragilis (ATCC25285) is known to have an activity of inducing IL-10, which is an anti-inflammatory cytokine (Cell Host & Microbe 20, 535-547, 2016), the IL-10-inducing ability of the intestinal bacteria, which were isolated this time, for bone marrow-derived dendritic cells (hereinafter also abbreviated as BMDCs) was evaluated.

<Test Method>

(Preparation of Mouse-derived BMDCs)

Bone marrow cells were extracted from a femur and a tibia of BALB/cAJcl mice and were suspended in a medium shown in Table 1 such that the final concentration was $3 \times 10^5$ cells/ml. GM-CSF (PeproTech) of 20 ng/ml was added, and the cells were cultured for 7 days to induce BMDCs. The induced BMDCs were subjected to antibody staining with CD11c-PE (Biolegend), and the proportion of CD11c-positive cells was confirmed using a flow cytometer. The cells in which the proportion of CD11c-positive cells was 80% or more were used as the BMDCs for evaluation. The BMDCs were cryopreserved at -150°C until they were subjected to co-culture.

[Table 1]

| Volume | Reagents | |
|--------|----------|---|
| 5 ml | Penicillin-Streptomycin-Glutamine (100X), Liquid (GIBCO: 10378-016) | 1X |
| 5 ml | HEPES, 1M Buffer Solution (GIBCO: 15630-080) | 10 mM |
| 50 ml | FBS (GIBCO inactivation, completed sterilization by filtration) | 10% |
| 440 ml | RPMI 1640 (GIBCO: 11875-093) | |

(Preparation of Bacteria Sample for Evaluation)

Veillonella sp. (JKB232V) and Alistipes putredinis (JKB232A) were subjected to static culture in an EG liquid medium containing blood at 37°C for 3 days. The obtained culture solution was centrifuged (at 2500 rpm for 10 minutes), and then the supernatant was collected and passed through a 0.2 μm PVDF filter (Merck) to obtain an evaluation sample of the supernatant. Subsequently, the bacteria cells were prepared with PBS so as to form a 10% bacteria cell suspension, followed by performing a heat treatment at 80°C for 20 minutes, and dead bacteria cells were prepared. The supernatant and the dead bacteria cells were stored at -30°C until they were subjected to co-culture. On the day when co-culture is performed, dead bacteria cells were thawed and prepared with PBS such that the ratio of the BMDCs to the intestinal bacteria (ratio of cells:dead bacteria cells) was 1:500, 1:150, or 1:50, and evaluation samples of dead bacteria cells were prepared.

(Co-culture Conditions)

On the day when the co-culture is started, the BMDCs were thawed and suspended at $2.2 \times 10^5$ cells/ml in a medium shown in Table 2, and then the suspension was dispensed into a 96-well plate in an amount of 90 $\mu$l per well. The evaluation sample was added in an amount of 10 $\mu$l per well, and culture was started under conditions of a liquid amount of 100 $\mu$L (PBS 10%), 37°C, and 5% $CO_2$. The culture was performed in the case of n = 3. As a positive control, LPS-EB Ultrapure (InvivoGen) was used. As a negative control, a sample-free medium was used. The final concentration was 1000 ng/ml. After 24 hours of culture, a supernatant was collected and cryopreserved at -30°C until the measurement.

[Table 2]

| Volume | Reagents | |
| --- | --- | --- |
| 5 ml | Penicillin-Streptomycin-Glutamine (100X), Liquid (GIBCO: 10378-016) | 1X |
| 5 ml | HEPES, 1M Buffer Solution (GIBCO: 15630-080) | 10 mM |
| 50 ml | FBS (GIBCO inactivation, completed sterilization by filtration) | 10% |
| 440 ml | DMEM (GIBCO: 11995-065) | |

(IL-10 Measurement)

Kit name: IL-10 (mouse) AlphaLISA Detection Kit (Perkin Elmer)
Number of measurements: 4 wells were used per sample.
Range of calibration curve: the top concentration of IL-10 was 100000 pg/ml, and 9 steps of dilution series were prepared by 3-fold dilution.
Measurement instrument: absorbance value measurement (450 nm) was performed using "Envision" manufactured by PerkinElmer.

<Results>

(Test 1)

IL-10-inducing activities of Veillonella sp. (JKB232V) and Alistipes putredinis (JKB232A) isolated were evaluated when each of Veillonella sp. (JKB232V) and Alistipes putredinis (JKB232A) isolated was subjected to co-culture such that a ratio of BMDCs to intestinal bacteria (ratio of cells:dead bacteria cells) was 1:500, 1:150, or 1:50. The results are shown in FIG. 1.
As shown in FIG. 1, regarding Veillonella sp. (JKB232V) and Alistipes putredinis (JKB232A), a high IL-10-inducing activity of 3000 pg/ml or more was observed when the ratio of the BMDCs to the intestinal bacteria (ratio of cells:dead bacteria cells) was 1:500. In particular, a high induction activity equal to or higher than that of LPS was observed in Veillonella sp. (JKB232V) even in the case of a small amount of intestinal bacteria (1:50).

(Test 2)

IL-10-inducing activities of Veillonella sp. (JKB232V) and Alistipes putredinis (JKB232A) were evaluated when a culture solution supernatant of each of Veillonella sp. (JKB232V) and Alistipes putredinis (JKB232A) isolated was co-cultured with BMDCs. The results are shown in FIG. 2.
As shown in FIG. 2, a high IL-10-inducing activity equal to or higher than that of LPS was also observed when the supernatant of each of Veillonella sp. (JKB232V) and Alistipes putredinis (JKB232A) was co-cultured with the BMDCs.

[Example3] Evaluation of IL-22-inducing Activity for Mouse BMDCs of Isolated Intestinal Bacteria

<Test Method>

Mouse-derived BMDCs were prepared in the same manner as in Example 1. Veilonella sp. (JKB232V) was subjected to static culture in an EG liquid medium containing blood at 37°C for 3 days. The obtained culture solution was centrifuged (at 2500 rpm for 10 minutes), and then a supernatant was discarded to prepare a 10% bacteria cell suspension with PBS. The bacteria cell suspension was heated at 80°C for 20 minutes to prepare dead bacteria cells. The dead bacteria cells were stored at -30°C until they were subjected to co-culture. On the day when co-culture is performed, dead bacteria cells were thawed and prepared with PBS such that the ratio of the BMDCs to the intestinal bacteria (ratio of cells:dead bacteria cells) was 1:500, and evaluation samples were prepared.

(Co-culture Conditions)

On the day when the co-culture is started, the BMDCs were thawed and suspended at $2 \times 10^5$ cells/ml in a medium shown in Table 2, and then the suspension was dispensed into a 96-well plate in an amount of 90 $\mu$L per well. The evaluation sample was added in an amount of 10 $\mu$L per well, and culture was started under conditions of a liquid amount of 100 $\mu$L (PBS 10%), 37°C, and 5% $CO_2$. The culture was performed in the case of n = 3. As a positive control, LPS-EB Ultrapure (InvivoGen) was used, and the final concentration of the positive control was 1000 ng/ml. As a negative control, a sample-free medium was used. After 24 hours of culture, a supernatant was collected and cryopreserved at -30°C until the measurement.

(Measurement of IL-22 Using ELISA Method)

Kit name: Mouse/Rat IL-22 Quantkine ELISA (R&D)
Number of measurements: 1 well was used per sample.
Dilution ratio: not diluted
Range of calibration curve: the top concentration of IL-22 was 1000 pg/ml, and 7 steps of dilution series were prepared by 2-fold dilution.
Measurement instrument: absorbance value measurement (450 nm) was performed using "Envision" manufactured by PerkinElmer.

<Results>

As shown in FIG. 3, an IL-22-inducing activity higher than that of LPS was observed in the dead bacteria cells of Veillonella sp. (JKB232V).

[Example 4] Evaluation 1 of Cytokine-IL-10-inducing Activity Enhancing Effect Caused by Veillonella sp.

The IL-10-inducing activity enhancing effect caused by a combination of Veillonella sp. (JKB232V) and an intestinal bacterium belonging to Bacteroidetes was evaluated.

<Test Method>

The preparation of the mouse-derived BMDCs and the measurement of IL-10 were performed in the same manner as in Example 1.

(Preparation of Bacteria Sample for Evaluation)

The single bacteria cell of Veillonella sp. (JKB232V), Alistipes putredinis (JKB232A), Bacteroides cellulosilyticus (JKB161), Bacteroides intestinalis (JKB231), or Bacteroides dorei (JKB233), or a bacteria mixture obtained by mixing any one of Alistipes putredinis (JKB232A), Bacteroides cellulosilyticus (JKB161), Bacteroides intestinalis (JKB231), and Bacteroides dorei (JKB233) with Veillonella sp. (JKB232V) at a ratio of approximately 1:1 was subjected to static culture in an EG liquid medium containing blood at 37°C for 3 days to 7 days. The obtained culture solution was centrifuged (at 2500 rpm for 10 minutes), and then a supernatant was discarded to prepare a bacteria cell suspension with PBS so as to have a bacteria cell concentration of 10%. The bacteria cell suspension was heated at 80°C for 20 minutes to prepare dead bacteria cells. The dead bacteria cells were stored at -30°C until they were subjected to co-culture. On the day when co-culture is performed, dead bacteria cells were thawed and prepared with PBS such that the ratio of the BMDCs to the intestinal bacteria (ratio of cells:dead bacteria cells) was 1:150, and evaluation samples of the dead bacteria cells were prepared.

(Co-culture Conditions)

On the day when the co-culture is started, the BMDCs were thawed and suspended at $2.2 \times 10^5$ cells/ml in a medium shown in Table 2, and then the suspension was dispensed into a 96-well plate in an amount of 90 µl per well. The evaluation sample was added in an amount of 10 µl per well, and culture was started under conditions of a liquid amount of 100 µL (PBS 10%), 37°C, and 5% $CO_2$. The culture was performed in the case of n = 3. As a positive control, LPS-EB Ultrapure (InvivoGen) was used, and the final concentration of the positive control was 1000 ng/ml. As a negative control, a sample-free medium was used. After 24 hours of culture, a supernatant was collected and cryopreserved at -30°C until the measurement.

(Calculation of Activity Value) The IL-10-inducing activity was calculated from the following formula 1, assuming that the concentration of IL-10 produced from the mouse-derived

BMDCs when the mouse-derived BMDCs was stimulated with 1000 ng/ml of LPS was defined as 100%. In the formula 1, the S value represents a concentration of IL-10 produced when stimulation is performed with an evaluation sample, the M value represents a concentration of IL-10 produced when stimulation is performed with a medium, and the L value represents a concentration of IL-10 produced when stimulation is performed with LPS.

$$\text{(Formula 1) (S value - M value)} \div \text{(L value - M value)} \times 100 = \text{Activity value (\%)}$$

<Results>

(Test 1)

As shown in FIGS. 4 and 5, as compared with a case where each of Alistipes putredinis (JKB232A), Bacteroides cellulosilyticus (JKB161), Bacteroides intestinalis (JKB231), and Bacteroides dorei (JKB233) is cultured alone, a higher IL-10-inducing activity was observed when each of the above bacteria was subjected to mixed culture with Veillonella sp. (JKB232V). In particular, with the mixed culture with Veillonella sp. (JKB232V), an enhancement in the IL-10-inducing activity of Alistipes putredinis (JKB232A) of about 3 times was observed.

[Example 5] Evaluation 2 of Cytokine-IL-10-inducing Activity Enhancing Effect Caused by Veillonella sp.

Regarding a mixture of dead bacteria cells of Veillonella sp. (JKB232V) and dead bacteria cells of an intestinal bacterium belonging to Bacteroidetes, the IL-10-inducing activity enhancing effect was evaluated. The preparation of the mouse-derived BMDCs and the measurement of IL-10 were performed in the same manner as in Example 1.

(Preparation of Bacteria Sample for Evaluation)

Each of Veillonella sp. (JKB232V), Alistipes putredinis (JKB232A), Bacteroides cellulosilyticus (JKB161), Bacteroides intestinalis (JKB231), and Bacteroides dorei (JKB233) was subjected to static culture in an EG liquid medium containing blood at 37°C for 3 days to 7 days. The obtained culture solution was centrifuged (at 2500 rpm for 10 minutes), and then a supernatant was discarded to prepare a bacteria cell suspension with PBS so as to have a bacteria cell concentration of 10%. The bacteria cell suspension was heated at 80°C for 20 minutes to prepare dead bacteria cells. The dead bacteria cells were stored at -30°C until they were subjected to co-culture.

The dead bacteria cells were thawed on the day when co-culture is performed, the dead bacteria cells of Veillonella sp. (JKB232V) were mixed with dead bacteria cells of each of Alistipes putredinis (JKB232A), Bacteroides cellulosilyticus (JKB161), Bacteroides intestinalis (JKB231), and Bacteroides dorei (JKB233) at a ratio of 0:1, 9:1, 4:1, 1:1, 1:4, or 1:9, then the mixture of the dead bacteria cells was prepared with PBS such that the ratio of BMDCs to the intestinal bacteria (ratio of cells:dead bacteria cells) was 1:500, and an evaluation sample was prepared.

(Co-culture Conditions)

On the day when the co-culture is started, the BMDCs were thawed and suspended at $2.2 \times 10^5$ cells/ml in a medium shown in Table 2, and then the suspension was dispensed into a 96-well plate in an amount of 90 µl per well. The evaluation sample was added in an amount of 10 µl per well, and culture was started under conditions of a liquid amount of 100 µL (PBS 10%), 37°C, and 5% $CO_2$. The culture was performed in the case of n = 3. As a positive control, LPS-EB Ultrapure (InvivoGen) was used, and the final concentration of the positive control was 1000 ng/ml. As a negative control, a sample-free medium was used. After 24 hours of culture, a supernatant was collected and cryopreserved at -30°C until the measurement.

As shown in FIGS. 6 and 7, as compared with the case where dead bacteria cells of each of Alistipes putredinis (JKB232A), Bacteroides cellulosilyticus (JKB161), Bacteroides intestinalis (JKB231), and Bacteroides dorei (JKB233) were co-cultured with the BMDCs alone, a higher IL-10-inducing activity was observed in the case where dead bacteria cells of Veillonella sp. (JKB232V) were mixed with the above dead bacteria cells thereof at a proportion of one-tenth (1:9), followed by co-culturing with the BMDCs. In particular, by mixing Veillonella sp. (JKB232V) and Bacteroides intestinalis (JKB231) at a ratio of 1:9, an enhancement in the IL-10-inducing activity about 1.4 times that of Bacteroides intestinalis (JKB231) alone was observed.

[Example 6] Evaluation of IL-10-Inducing Activity of Isolated Intestinal Bacteria for Human Peripheral Blood-derived Dendritic Cells

Regarding a single sample of Veillonella sp. (JKB232V) or a mixed culture sample of Alistipes putredinis (JKB232A) and Veillonella sp. (JKB232V) which exhibited a high IL-10-inducing activity for the mouse BMDCs in Examples 2 and 4, an IL-10-inducing ability for human peripheral blood-derived dendritic cells (hereinafter also abbreviated as DC) was evaluated.

<Test Method>

(Preparation of Human Peripheral Blood-derived Dendritic Cells)

CD14-positive cells were separated from commercially available human peripheral blood mononuclear cells (AllCells, catalog number LP, CR, MNC, 100M) using MACS (Miltenyi Biotec). The separated cells were subjected to antibody staining using CD14-FITC (Biolegend), and the proportion of CD14-positive cells was confirmed to be 94% or more using a flow cytometer. Next, the obtained CD14-positive cells were prepared to have a final concentration of $2 \times 10^6$ cells/ml using the medium shown in Table 4, followed by culturing in a medium obtained by 50 ng/ml of GM-CSF (R&D Systems) and 100 ng/ml of IL-4 (R&D Systems) were added to the composition shown in Table 1. The medium was replaced after 2 days and 4 days after the culture, and DC was induced by the culture for 7 days in total. The induced DC was subjected to antibody staining using PerCP/Cy5.5 anti-human HLA-DR Antibody (Biolegend) or CD11c PE cy7 (Sony Biotechnology), and HLA-DR-positive cells and CD11c-positive cells were confirmed using a flow cytometer, and DC for evaluation was prepared.

(Preparation of Bacteria Sample for Evaluation)

The isolated Veillonella sp. (JKB232V) or mixed bacteria cells obtained by mixing Alistipes putredinis (JKB232A) and Veillonella sp. (JKB232V) at a ratio of approximately 1:1 were subjected to static culture in an EG liquid medium containing blood at 37°C for 3 days to 7 days. The obtained culture solution was centrifuged (at 2500 rpm for 10 minutes), and then a supernatant was discarded to prepare a 10% bacteria cell suspension with PBS. The bacteria cell suspension was heated at 80°C for 20 minutes to prepare dead bacteria cells, and then the dead bacteria cells were stored at -30°C until they were subjected to co-culture. On the day when co-culture is performed, dead bacteria cells were thawed and prepared with PBS such that the ratio of the DCs to the intestinal bacteria (ratio of cells:dead bacteria cells) was 1:500, and evaluation samples of dead bacteria cells were prepared.

(Co-culture Conditions)

With respect to 1 well of a culture plate, 90 µl of the DCs prepared with the medium shown in Table 1 so as to have a final concentration of $1.2 \times 10^5$ cells/ml (Lot.A) or $6.7 \times 10^4$ cells/ml (Lot.B) was dispensed into each well. As the culture plate, a 96-well flat bottom plate manufactured by FALCON was used. After culture for about 3 hours under the conditions of 37°C and 5% $CO_2$, 10 µl of an evaluation sample was added. The culture was performed in the case of n = 2. As a positive control, LPS-EB Ultrapure (InvivoGen) was used, and the final concentration of the positive control was 1000 ng/ml. As a negative control, a sample-free medium was used. After culture for 24 hours, the supernatant was collected and cryopreserved at -30°C.

(Measurement of IL-10 Using AlphaLISA Method)

Kit name: Human IL-10 AlphaLISA Detection Kit (PerkinElmer)
Number of measurements: 4 wells were used per sample.
Dilution ratio: measurement was performed by diluting the culture supernatant by 2 times.
Range of calibration curve: the top concentration of IL-10 was 10000 pg/ml, and 8 steps of dilution series were prepared up to 3 pg/ml.
Measurement: using "Envision" manufactured by PerkinEliner, measurement was performed according to the instruction manual of the kit.

<Results>

As shown in FIG. 8, a high IL-10-inducing activity of 3000 pg/ml or more was observed in the dead bacteria cells of Veillonella sp. (JKB232V), and a high IL-10-inducing activity of 6000 pg/ml or more was observed in the dead bacteria cells obtained by the mixed culture of Alistipes putredinis (JKB232A) and Veillonella sp. (JKB232V).

[Example 7] Evaluation of Cytokine-IL-22-inducing Activity of Isolated Intestinal Bacteria

The IL-22-inducing activity enhancing effect caused by Veillonella sp. (JKB232V) alone or a combination of Veillonella sp. (JKB232V) and an intestinal bacterium belonging to Bacteroidetes was evaluated.

<Test Method>

(Preparation of Mouse-derived BMDCs)

Bone marrow-derived mouse precursor cells of BALB/c mice (manufactured by Cosmo Bio Co., Ltd.) were suspended in a dendritic cell differentiation medium DCDM (manufactured by Cosmo Bio Co., Ltd.) and cultured at 37°C for 8 days to induce the BMDCs. The induced BMDCs were collected, followed by counting the number of cells with an automatic cell counter Countess II FL (manufactured by Invitogen), and the BMDCs were prepared using the DCDM to reach $2 \times 10^6$ cells/ml.

(Preparation of Bacteria Sample for Evaluation)

Each of Veillonella sp. (JKB232V), Bacteroides cellulosilyticus (JKB161), Bacteroides intestinalis (JKB231), Alistipes putredinis (JKB232A), and Bacteroides dorei (JKB233) isolated in Example 1 was subjected to static culture in an EG liquid medium containing blood at 37°C for 3 days to 7 days. The obtained culture solution was centrifuged (at 2500 rpm for 10 minutes), followed by removing a supernatant and adjusting the concentration of the bacteria cells with PBS so as to obtain a 10% bacteria cell suspension, and dead bacteria cells were prepared by a heat treatment at 80°C for 20 minutes. The obtained dead bacteria cells of Veillonella sp. (JKB232V) and one kind of dead bacteria cells of each Bacteroidetes bacterium were mixed at a ratio of 1:1 to obtain mixed dead bacteria cells. The respective dead bacteria cells were stored at -80°C until they were subjected to co-culture. On the day when co-culture is performed, dead bacteria cells were thawed at 4°C and prepared with PBS such that the ratio of the BMDCs to the intestinal bacteria (ratio of cells:dead bacteria cells or mixed dead bacteria cells) was 1:500, and evaluation samples of dead bacteria cells were prepared.

(Co-culture Conditions)

The prepared BMDCs were dispensed into a 96-well plate in an amount of 100 μL per well. Subsequently, a mixed solution of 10 μL of the evaluation sample and 90 μL of PBS was added to a well, and culture was started under conditions of a total amount of 200 μL per well, 37°C, and 5% $CO_2$. The culture was performed in the case of n = 3. As a positive control, LPS E coli.O111: B4 (manufactured by Sigma-Aldrich) was used, and the final concentration of the positive control was 1000 ng/ml. As a negative control, a sample-free medium was used. After 24 hours of culture, a supernatant was collected and cryopreserved at -80°C until the measurement.

(Measurement of IL-22 Using ELISA Method)

Kit name: Mouse/Rat IL-22 Quantkine ELISA (R&D)
Number of measurements: 1 well was used per sample.
Dilution ratio: no
Range of calibration curve: the top concentration of IL-22 was 1000 pg/ml, and 7 steps of dilution series were prepared by 2-fold dilution.
Measurement instrument: absorbance value measurement (450 nm) was performed using Varuiskan Lux (manufactured by Thermofisher scienticfic).

<Results>

As shown in FIG. 9, an IL-22-inducing activity higher than that of LPS was observed in the dead bacteria cells of Veillonella sp. (JKB232V). In addition, as compared with an IL-22-inducing activity of respective dead bacteria cells of Bacteroides cellulosilyticus (JKB 161), Bacteroides intestinalis (JKB231), Alistipes putredinis (JKB232A), and Bacteroides dorei (JKB233), a higher IL-22-inducing activity was exhibited by combining the above respective dead bacteria cells thereof with dead bacteria cells of Veillonella sp. (JKB232V).

Although the present invention has been described in detail with reference to specific embodiments, it will be apparent to those skilled in the art that various changes and modifications can be made without departing from the spirit and scope of the invention. The present application is based on a Japanese Patent Application No. 2021-036484 filed on March 8, 2021, the entire contents of which are incorporated herein by reference. All references cited herein are incorporated in their entirety.

**Claims**

1. A composition comprising an intestinal bacterium belonging to the genus Veillonella and having an inflammation reducing activity or a substance derived from the intestinal bacterium and having an inflammation reducing activity, the composition having at least one activity selected from an anti-inflammatory activity, an immunoregulation activity, an epithelial barrier restoring activity, an IL-10-inducing activity, and an IL-22-inducing activity.

2. The composition according to claim 1, wherein the intestinal bacterium is Veillonella sp. (JKB232V).

3. The composition according to claim 1 or 2, further comprising an intestinal bacterium belonging to Bacteroidetes and having an inflammation reducing activity, or a substance derived from the intestinal bacterium and having an inflammation reducing activity.

4. The composition according to claim 3, wherein the intestinal bacterium belonging to Bacteroidetes is Alistipes putredinis, Bacteroides cellulosilyticus, Bacteroides intestinalis, or Bacteroides dorei.

5. The composition according to claim 4, wherein the above Alistipes putredinis, Bacteroides cellulosilyticus, Bacteroides intestinalis, and Bacteroides dorei are Alistipes putredinis (JKB232A), Bacteroides cellulosilyticus (JKB161), Bacteroides intestinalis (JKB231), and Bacteroides dorei (JKB233), respectively.

6. An ameliorating agent for an inflammatory disease, an autoimmune disease, and an infectious disease, which contains an intestinal bacterium belonging to genus Veillonella and having an inflammation reducing activity or a substance derived from the intestinal bacterium and having an inflammation reducing activity, and which has at least one activity selected from an anti-inflammatory activity, an immunoregulation activity, an epithelial barrier restoring activity, an IL-10-inducing activity, and an IL-22-inducing activity.

7. The ameliorating agent according to claim 6, wherein the intestinal bacterium is Veillonella sp.(JKB232V).

8. The ameliorating agent according to claim 6 or 7, further comprising an intestinal bacterium belonging to Bacteroidetes and having an inflammation reducing activity, or a substance derived from the intestinal bacterium and having an inflammation reducing activity.

9. The ameliorating agent according to claim 8, wherein the intestinal bacterium belonging to Bacteroidetes is Alistipes putredinis, Bacteroides cellulosilyticus, Bacteroides intestinalis, or Bacteroides dorei.

10. The ameliorating agent according to claim 9, wherein the above Alistipes putredinis, Bacteroides cellulosilyticus, Bacteroides intestinalis, and Bacteroides dorei are Alistipes putredinis (JKB232A), Bacteroides cellulosilyticus (JKB161), Bacteroides intestinalis (JKB231), and Bacteroides dorei (JKB233), respectively.

11. The ameliorating agent according to any one of claims 6 to 10, wherein the inflammatory disease, the autoimmune disease, or the infectious disease is at least one selected from the group consisting of an inflammatory bowel disease, an allergic disease, an infectious disease, diabetes, multiple sclerosis, rheumatoid arthritis, and a graft-versus-host rejection response.

12. A pharmaceutical product comprising the ameliorating agent according to any one of claims 6 to 11.

13. A food comprising the ameliorating agent according to any one of claims 6 to 11.

14. A microorganism that is Veillonella sp. (JKB232V).

15. An method for ameliorating an inflammatory disease, an autoimmune disease, or an infectious disease, the method comprising applying an intestinal bacterium belonging to the genus Veillonella and having an inflammation reducing activity or a substance derived from the intestinal bacterium and having an inflammation reducing activity, and the intestinal bacterium or the substance having at least one activity selected from an anti-inflammatory activity, an immunoregulation activity, an epithelial barrier restoring activity, an IL-10-inducing activity, and an IL-22-inducing activity.

16. Use of an intestinal bacterium belonging to the genus Veillonella and having an inflammation reducing activity or a substance derived from the intestinal bacterium and having an inflammation reducing activity for the manufacture of an ameliorating agent for an inflammatory disease, an autoimmune disease, and an infectious disease which has at least one activity selected from an anti-inflammatory activity, an immunoregulation activity, an epithelial barrier restoring activity, an IL-10-inducing activity, and an IL-22-inducing activity.

17. An intestinal bacterium belonging to the genus Veillonella and having an inflammation reducing activity or a substance derived from the intestinal bacterium and having an inflammation reducing activity for use as an ameliorating agent for an inflammatory disease, an autoimmune disease, and an infectious disease which has at least one activity selected from an anti-inflammatory activity, an immunoregulation activity, an epithelial barrier restoring activity, an IL-10-inducing activity, and an IL-22-inducing activity.

## FIG. 1

## FIG. 2

## FIG. 3

## FIG. 4

## FIG. 5

## FIG. 6

## FIG. 7

## FIG. 8

## FIG. 9

## INTERNATIONAL SEARCH REPORT

| International application No. |
|---|
| **PCT/JP2022/009997** |

**A. CLASSIFICATION OF SUBJECT MATTER**

*A61K 35/74*(2015.01)i; *A23L 33/135*(2016.01)i; *A61P 1/04*(2006.01)i; *A61P 3/10*(2006.01)i; *A61P 17/00*(2006.01)i; *A61P 19/02*(2006.01)i; *A61P 25/00*(2006.01)i; *A61P 29/00*(2006.01)i; *A61P 31/00*(2006.01)i; *A61P 37/02*(2006.01)i; *A61P 37/06*(2006.01)i; *A61P 37/08*(2006.01)i; *A61P 43/00*(2006.01)i; *C12N 1/20*(2006.01)i

FI: A61K35/74 A; A23L33/135; A61K35/74 G; A61P1/04; A61P3/10; A61P17/00; A61P19/02; A61P25/00; A61P29/00; A61P29/00 101; A61P31/00; A61P37/02; A61P37/06; A61P37/08; A61P43/00 111; A61P43/00 121; C12N1/20 A

According to International Patent Classification (IPC) or to both national classification and IPC

**B. FIELDS SEARCHED**

Minimum documentation searched (classification system followed by classification symbols)

A61K35/74; A23L33/135; A61P1/04; A61P3/10; A61P17/00; A61P19/02; A61P25/00; A61P31/00; A61P37/02; A61P37/06; A61P37/08; A61P43/00; C12N1/20

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/157003 A1 (EVELO BIOSCIENCES, INC.) 15 August 2019 (2019-08-15) particularly, claims, examples 2, 24, fig. 9, paragraphs [0114], [0141], [0012], [0115], [0050] | 1, 2, 6, 7, 11-17 |
| Y | particularly, claims, examples 2, 24, fig. 9, paragraphs [0114], [0141], [0012], [0115], [0050] | 3-5, 8-13 |
| Y | NEFF, C. Preston et al. Diverse Intestinal Bacteria Contain Putative Zwitterionic Capsular Polysaccharides with Anti-inflammatory Properties. Cell Host Microbe. 2016, vol. 20, no. 4, pages 535-547, doi: 10.1016/j.chom.2016.09.002 particularly, abstract, fig. 4, 5, table 1 | 3-5, 8-13 |
| Y | CHANG, Yi-Chih et al. TLR2 and interleukin-10 are involved in Bacteroides fragilis-mediated prevention of DSS-induced colitis in gnotobiotic mice. PLoS One. 2017, vol. 12, no. 7, article.e0180025 (pp. 1-16), doi: 10.1371/journal.pone.0180025 particularly, abstract, fig. 7, 8, table 2, etc. | 3-5, 8-13 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 April 2022** | **17 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/009997**

C.     DOCUMENTS CONSIDERED TO BE RELEVANT

| Category* | Citation of document, with indication, where appropriate, of the relevant passages | Relevant to claim No. |
|---|---|---|
| Y | SCHIRMER, Melanie et al. Dynamics of metatranscription in the inflammatory bowel disease gut microbiome. Nature Microbiology. 2018, vol. 3, no. 3, pages 337-346, DOI: 10.1038/s41564-017-0089-z<br>particularly, abstract, page 7, fig. 4, etc. | 3-5, 8-13 |

Form PCT/ISA/210 (second sheet) (January 2015)

## INTERNATIONAL SEARCH REPORT
### Information on patent family members

| International application No. |
| --- |
| **PCT/JP2022/009997** |

| Patent document cited in search report | Publication date (day/month/year) | Patent family member(s) | Publication date (day/month/year) |
| --- | --- | --- | --- |
| WO 2019/157003 A1 | 15 August 2019 | JP 2021-512133 A<br>particularly, claims, examples 2, 24, fig. 9, paragraphs [0102], [0129], [0012], [0100], [0036]<br>US 2019/0350987 A<br>EP 3749340 A<br>TW 201945015 A<br>KR 10-2020-0118100 A<br>CN 112074283 A<br>AU 2019217526 A<br>AR 114246 A<br>CA 3090166 A<br>BR 112020015639 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016102950 A
- US 6368586 B
- JP 2021036484 A

**Non-patent literature cited in the description**

- **ISHIKAWA et al.** *Inflamm Bowel Dis.,* 2018, vol. 24, 2590-2598
- **ATARASHI et al.** *Science,* 2011, vol. 331, 337-341
- **ATARASHI et al.** *Nature,* 2013, vol. 500, 232-238
- **DELDAY et al.** *Inflamm Bowel Dis.,* 2019, vol. 25, 85-96
- **IHEKWEAZU et al.** *GUT MICROBES,* 2019, vol. 10, 504-520
- **WAYNE et al.** *Int. J. Syst. Bacteriol.,* 1987, vol. 37, 463-464
- **GORIS, J. et al.** *Int. J. Syst. Evol. Microbiol.,* 2007, vol. 57, 81-91
- **RICHTER, M. ; ROSSELLO-MORA, R.** *Proc. Natl. Acad. Sci. USA,* 2009, vol. 106, 19126-19131
- **SRUTKOVA et al.** *J. Microbiol.Methods,* 2011, vol. 87 (1), 10-6
- **FRANKE A et al.** *Nat Genet.,* 2008, vol. 40, 1319-23
- *Cell Host & Microbe,* 2016, vol. 20, 535-547